# EUROPÄISCHE PATENTANMELDUNG

(11) **EP 4 468 303 A1**
(43) Veröffentlichungstag der Anmeldung: **27.11.2024**
(21) Anmeldenummer: 24177818.2
(22) Anmeldetag: 24.05.2024
(51) Int. Cl.: G16H 20/17, G16H 40/63, A61M 1/14

(54) **VERFAHREN ZUM BETREIBEN EINES DIALYSESYSTEMS**

(30) Priorität: 24.05.2023 DE 102023113670
(71) Anmelder: B. Braun Avitum AG, 34212 Melsungen (DE)
(72) Erfinder: KRIETEMEYER, Stephan, 34212 Melsungen (DE); ODERNHEIMER, Philipp, 34212 Melsungen (DE); LIEB, Nino, 34212 Melsungen (DE); GIESA, Jonas, 34212 Melsungen (DE)
(74) Vertreter: Maiwald GmbH

(57) **Zusammenfassung**

Die Erfindung betrifft ein Verfahren zum Betreiben eines Dialysesystems umfassend mehrere hydraulisch miteinander in Verbindung stehende Komponenten und eine Steuervorrichtung, wobei das Verfahren umfasst: Empfangen von Komponenten-Betriebsdaten von den Komponenten, wobei die Komponenten-Betriebsdaten zumindest Bedarfsdaten für eine bevorstehende, durch die jeweilige Komponente auszuführende Aufgabe umfassen, Bestimmen eines zu erwartenden Ressourcenverbrauchs der Komponente anhand der Bedarfsdaten, Überwachung eines tatsächlichen Ressourcenverbrauchs der Komponenten, Bestimmen einer Abweichung des tatsächlichen Ressourcenverbrauchs von dem zu erwartenden Ressourcenverbrauch, und Erkennen einer Leckage im Dialysesystem, wenn die Abweichung außerhalb eines vorgegebenen Bereichs liegt.

## Beschreibung

Offenbart sind ein Verfahren zum Betreiben eines Dialysesystems, ein Dialysesystem, ein System umfassend ein Dialysesystem, ein Computerprogrammprodukt und ein computerlesbares Medium.

Bislang stehen verschiedene Komponenten von Dialysesystemen, hier auch als Medizinprodukte bezeichnet, nur in hydraulischer Verbindung miteinander und werden einzeln und in der Regel manuell programmiert. Bei solchen Systemen kann Leckage im Gesamtsystem nicht zuverlässig erkannt werden.

Eine Aufgabe, die der Erfindung zugrunde liegt, besteht also darin, ein Verfahren zum Betreiben eines Dialysesystems bereitzustellen, das Leckage zuverlässig erkennt.

Die Aufgabe wird durch den Gegenstand der unabhängigen Ansprüche gelöst. Bevorzugte Ausführungsformen finden sich in den abhängigen Ansprüchen.

Die Erfindung betrifft ein Verfahren zum Betreiben eines Dialysesystems umfassend mehrere hydraulisch miteinander in Verbindung stehende Komponenten und eine Steuervorrichtung. Das Verfahren umfasst ein Empfangen, durch die Steuervorrichtung, von Komponenten-Betriebsdaten von den Komponenten, wobei die Komponenten-Betriebsdaten zumindest Bedarfsdaten für eine bevorstehende, durch die jeweilige Komponente auszuführende Aufgabe umfassen. Das Verfahren umfasst auch ein Bestimmen eines zu erwartenden Ressourcenverbrauchs der (jeweiligen) Komponente anhand der Bedarfsdaten. Dabei kann es sich beispielsweise um den zu erwartenden Ressourcenverbrauch für die bevorstehende, durch die Komponente auszuführende Aufgabe handeln. Das Verfahren umfasst auch eine Überwachung, durch die Steuervorrichtung, eines tatsächlichen Ressourcenverbrauchs der Komponenten, ein Bestimmen, durch die Steuervorrichtung, einer Abweichung des tatsächlichen Ressourcenverbrauchs, von dem zu erwartenden Ressourcenverbrauch, und ein Erkennen, durch die Steuervorrichtung, einer Leckage im Dialysesystem, wenn die Abweichung außerhalb eines vorgegebenen Bereichs liegt. Die (jeweiligen) Betriebsdaten können dabei von einer oder mehreren der in Verbindung stehenden Komponenten, insbesondere von allen der in Verbindung stehenden Komponenten, empfangen werden.

So kann eine Leckage im Dialysesystem zuverlässig erkannt werden. Bei bekannten Systemen ist diese Art der Leckage-Erkennung, insbesondere auf Systemebene, nicht möglich. Eine Leckage kann bestenfalls an Einzelkomponenten durch individuelle Überwachung der jeweiligen Komponente erfolgen, was aber in manchen Fällen schwierig oder nicht möglich ist.

Das Dialysesystem kann beispielsweise Teil eines Dialysezentrums oder einer Dialysepraxis sein.

Die Komponenten, auch als Medizinprodukte bezeichnet, können beispielsweise Dialysegeräte, Heißreinigungsvorrichtungen, Umkehrosmosegeräte, Konzentratmischanlagen oder dergleichen umfassen.

Dass die Komponenten hydraulisch miteinander in Verbindung stehen bedeutet, dass Flüssigkeit zwischen den Komponenten gefördert, beispielsweise gepumpt, werden kann. Die hydraulische Verbindung kann in Form von Schläuchen und/oder Rohren ausgebildet sein. Die hydraulische Verbindung kann direkt oder indirekt sein. Das Dialysesystem kann Ventile umfassen, die beispielsweise zum Einstellen eines Flusses durch das Dialysesystem verwendet werden können, beispielsweise automatisch ansteuerbare Ventile.

Die Steuervorrichtung kann integral mit einer der Komponenten ausgebildet sein oder separat von den Komponenten ausgebildet sein.

Die Steuervorrichtung kann zum Empfangen von Daten von den Komponenten mittels einer, insbesondere bi-direktionalen, Datenverbindung mit der jeweiligen Komponente ausgebildet sein. Die Datenverbindung kann drahtlos oder drahtgebunden sein.

Der Begriff Komponenten-Betriebsdaten ist breit zu verstehen und kann beispielsweise Daten umfassen, die den Betrieb einer Komponente betreffen, so zum Beispiel Daten, die einen aktuellen und/oder geplanten Betriebsmodus der Komponente repräsentieren und/oder Daten, die die aktuellen und/oder geplanten Einstellungen von Betriebsparametern repräsentieren und/oder Daten, die den aktuellen Zustand der Komponente repräsentieren, beispielsweise Anzustand, Auzustand, oder Fehlerzustand. Die Komponenten-Betriebsdaten können auch ein zu erwartendes Ergebnis, beispielsweise einen erwarteten Output, umfassen.

Wie oben gesehen umfassen die Komponenten-Betriebsdaten zumindest Bedarfsdaten, die einen zu erwartenden Ressourcenverbrauch der (jeweiligen) Komponente für eine bevorstehende, durch die Komponente auszuführende Aufgabe angeben. Die Komponenten-Betriebsdaten können insbesondere die auszuführende Aufgabe angeben und den zugehörigen zu erwartenden Ressourcenverbrauch.

Die Bedarfsdaten können einen zu erwartenden Ressourcenverbrauch für eine oder mehrere Ressourcen angeben, beispielsweise einen Wasserverbrauch und einen Energieverbrauch. Die Bedarfsdaten müssen den zu erwartenden Ressourcenverbrauch nicht in einer bestimmten Darstellungsweise, beispielsweise bestimmten Einheiten, angeben. Es genügt, dass die Bedarfsdaten derart ausgebildet sind, dass anhand der Bedarfsdaten der Ressourcenverbrauch ableitbar ist, beispielsweise durch die Steuervorrichtung.

Das Maß für den Ressourcenverbrauch kann anwendungsbezogen definiert sein. So kann beispielsweise der Ressourcenverbrauch als Differenz zwischen Entnahmemenge und Abgabemenge, beispielsweise einströmenden und ausströmenden Volumen einer Flüssigkeit oder aufgenommener Wärme und Restwärme, oder als Entnahmemenge, beispielsweise Stromverbrauch, definiert werden.

Das Verfahren umfasst auch eine Überwachung, durch die Steuervorrichtung, eines tatsächlichen Ressourcenverbrauchs der (jeweiligen) Komponente.

Der tatsächliche Ressourcenverbrauch der Komponente kann beispielsweise anhand von Daten, die die Komponente selbst sammelt, bestimmt werden. Insbesondere kann die Komponente den Ressourcenverbrauch anhand dieser Daten selbst bestimmen.

Alternativ kann der tatsächlicher Ressourcenverbrauch der (jeweiligen) Komponente durch die Steuervorrichtung bestimmt werden. Dazu kann die Komponente die gesammelten Daten an die Steuervorrichtung übermitteln.

Der Ressourcenverbrauch kann auch anhand von Messdaten bestimmt werden, die nicht durch die (jeweilige) Komponente selbst gesammelt werden, sondern beispielsweise durch Sensoren, die nicht Teil der (jeweiligen) Komponente sind.

Die Sensoren können an der (jeweiligen) Komponente angeordnet sein. Beispielsweise können solche Sensoren Entnahmemenge und Abgabemenge einer Ressource bestimmen.

Zumindest ein Sensor, der Daten zum Bestimmen des Ressourcenverbrauchs einer Komponente sammeln, kann in anderen Bereichen des Systems, also nicht an der (jeweiligen) Komponente, angeordnet sein.

Beispielsweise kann ein Volumenstrom an verschiedenen Stellen im System gemessen werden und muss beispielsweise nicht unmittelbar am Eingang oder Ausgang einer Komponente gemessen werden.

Alternativ oder zusätzlich kann eine Komponente Daten sammeln, die zum Bestimmen des tatsächlichen Ressourcenverbrauchs einer anderen Komponente verwendet werden, beispielsweise indem davon ausgegangen wird, dass die bei einer Komponente gemessene Abgabemenge der Entnahmemenge einer anderen Komponente entspricht.

Beispielsweise kann, statt bei einer Komponente jeweils das einströmende und das ausströmende Volumen zu messen, auch jeweils nur das einströmende Volumen hydraulisch unmittelbar aufeinanderfolgender Komponenten gemessen werden. Wie oben bereits gesehen, kann das Maß für den Ressourcenverbrauch anwendungsbezogen geeignet definiert sein und die Messung kann entsprechend durchgeführt werden.

Die Überwachung des tatsächlichen Ressourcenverbrauchs der (jeweiligen) Komponente kann insbesondere eine kontinuierliche oder, beispielsweise in regelmäßigen Abständen, wiederholte Bestimmung des Ressourcenverbrauchs umfassen. Beispielsweise kann nach vorbestimmten Zeitintervallen der Ressourcenverbrauch bestimmt werden, entweder in Form eines aktuellen Messwertes oder integrierter Messwerte, insbesondere in Form von über das vorangegangene Zeitintervall integrierten Messwerten.

Das Verfahren umfasst auch ein Bestimmen, durch die Steuervorrichtung, einer Abweichung des tatsächlichen Ressourcenverbrauchs, von dem zu erwartenden Ressourcenverbrauch.

Das Bestimmen des tatsächlichen Ressourcenverbrauchs kann komponentenweise oder kollektiv für mehrere Komponenten erfolgen. Eine kollektive Bestimmung des Ressourcenverbrauchs ist unten im Zusammenhang mit einem beispielhaften Ringsystem, an das mehrere Komponenten angeschlossen sind, beschrieben.

Wenn der tatsächliche Ressourcenverbrauch kollektiv für mehrere Komponenten bestimmt wird, so versteht sich, dass als zu erwartender Ressourcenverbrauch ebenfalls der kollektive Ressourcenverbrauch für diese Komponenten zu betrachten ist.

Das Bestimmen der Abweichung, insbesondere das Quantifizieren der Abweichung, kann beispielsweise umfassen, dass eine Differenz und/oder ein Quotient aus tatsächlichem und zu erwartendem Ressourcenverbrauch bestimmt wird.

Die Abweichung kann dabei beispielsweise anhand der jeweiligen Abweichungen zwischen Einzelwerten des tatsächlichen und des zu erwartenden Ressourcenverbrauchs und/oder der Abweichung zwischen einem oder mehreren zeitlichen Mittelwerten des tatsächlichen und des zu erwartenden Ressourcenverbrauchs und/oder anhand der Abweichung zwischen einem oder mehreren integrierten bzw. kumulativen Werten des tatsächlichen und des zu erwartenden Ressourcenverbrauchs bestimmt werden.

Das Verfahren umfasst auch ein Erkennen, durch die Steuervorrichtung, einer Leckage im Dialysesystem, wenn die Abweichung außerhalb eines vorgegebenen Bereichs liegt.

Der Bereich kann als Toleranzbereich betrachtet werden. Eine bestimmte Abweichung kann beispielsweise auf Messfehler oder vernachlässigbare Verluste zurückgehen. Eine solche Abweichung wird aufgrund des Toleranzbereichs nicht unmittelbar dazu führen, dass eine Leckage erkannt wird. So werden falsch-positive Ereignisse reduziert.

Beispielsweise kann der vorgegebene Bereich durch einen oder mehrere vorbestimmte Grenzwerte definiert sein. Das Verfahren kann umfassen, dass eine Leckage erkannt wird, wenn ein vorbestimmter oberer Grenzwert überschritten wird und/oder wenn ein vorbestimmter unterer Grenzwert unterschritten wird.

Beispielsweise kann eine Leckage erkannt werden, wenn die Differenz aus tatsächlichem und zu erwartendem Ressourcenverbrauch einen oberen Grenzwert überschreitet.

Als ein anderes Beispiel kann eine Leckage erkannt werden, wenn ein Quotient aus zu erwartendem und tatsächlichem Ressourcenverbrauch einen unteren Grenzwert unterschreitet oder ein Quotient aus tatsächlichem und zu erwartendem Ressourcenverbrauch einen oberen Grenzwert überschreitet.

Eine Leckage kann unmittelbar, beispielsweise anhand einer Bilanzierung von Volumenströmen, erkannt werden. Allerdings wird eine Leckage in vielen Fällen auch den Verbrauch anderer Ressourcen beeinflussen, so dass eine mittelbare Leckage-Erkennung ebenfalls möglich ist. Beispielsweise kann eine übermäßig hohe Entnahme von Strom dafür sprechen, dass ein Druckabfall im System eine höhere Pumpleistung erforderlich macht. Der Druckabfall wiederum kann auf eine Leckage hindeuten.

Die Steuervorrichtung verwendet zusammenfassend die Bedarfsdaten, insbesondere den zu erwartenden Ressourcenverbrauch, und einen tatsächlichen Ressourcenverbrauch, um festzustellen, ob eine Leckage vorliegt. Die vorliegende Erfindung ermöglicht eine zuverlässige systemweite Überwachung und Erkennung von Leckagen.

Gemäß der vorliegenden Offenbarung kann der zu erwartende Ressourcenverbrauch durch einen oder mehrere Bedarfsparameter repräsentiert werden/sein und der tatsächliche Ressourcenverbrauch durch einen oder mehrere Verbrauchsparameter repräsentiert werden/sein, wobei jeweils ein Bedarfsparameter und ein Verbrauchsparameter ein Parameterpaar bilden und die Abweichung für mindestens eines der Parameterpaare bestimmt wird.

Ein Parameterpaar kann beispielsweise eine erwartete Entnahmemenge und eine tatsächliche und Entnahmemenge einer bestimmten Ressource, beispielsweise Flüssigkeitsvolumen, Wärmemenge oder Strom, umfassen. Ein weiteres Parameterpaar kann beispielsweise eine tatsächliche und eine erwartete Differenz aus einer Entnahmemenge und einer Abgabemenge einer Ressource, beispielsweise einer Flüssigkeit, umfassen.

Gemäß der vorliegenden Offenbarung kann für mehrere, insbesondere alle, Parameterpaare jeweils eine Abweichung des tatsächlichen Ressourcenverbrauchs von dem zu erwartenden Ressourcenverbrauch bestimmt werden und eine Leckage erkannt werden, wenn mindestens eine vorgegebene Anzahl der Abweichungen außerhalb eines für das Parameterpaar vorgegebenen Bereichs liegt.

Das heißt, eine Leckage kann nur dann festgestellt/erkannt werden, wenn mindestens für die vorgegebene Anzahl von Parameterpaaren eine Abweichung vorliegt, die für das Parameterpaar außerhalb des vorgegebenen Bereichs liegt, also nicht zulässig ist.

Der vorgegebene Bereich kann beispielsweise, wie oben erläutert, in Form von oberen und/oder unteren Grenzwerten vorgegeben sein.

Die vorgegebene Anzahl kann insbesondere größer oder gleich eins, insbesondere größer oder gleich zwei sein.

Beispielsweise kann die vorgegebene Anzahl eins sein. Das heißt, eine Leckage kann erkannt werden, wenn mindestens eine der Abweichungen außerhalb des für das Parameterpaar vorgegebenen Bereichs liegt. Dann kann also eine Leckage bereits dann erkannt werden, wenn für nur ein Parameterpaar die Abweichung außerhalb des vorgegebenen Bereichs liegt.

Dies ermöglicht eine besonders zuverlässige Erkennung aller möglichen Leckage-Ereignisse.

Alternativ kann die vorgegebene Anzahl größer eins sein, insbesondere zwei oder mehr. Dann kann beispielsweise nur dann eine Leckage erkannt werden, wenn mindestens für die vorgegebene Anzahl an Parameterpaaren die jeweilige Abweichung außerhalb des vorgegebenen Bereichs liegt, beispielsweise also für mindestens zwei Parameterpaare.

Durch eine vorgegebene Anzahl größer eins kann das Auftreten falsch-positiver Leckage-Erkennungen reduziert werden. Beispielsweise kann eine Abweichung für ein Parameterpaar auf einen Messfehler zurückgehen. Wird noch ein weiteres Parameterpaar herangezogen, so kann vermieden werden, dass aufgrund des Messfehlers eine falsch-positive Leckage-Erkennung erfolgt.

Gemäß der vorliegenden Offenbarung können die Komponenten einen oder mehrere Verbraucher umfassen und die Überwachung des tatsächlichen Ressourcenverbrauchs umfassen, dass die Steuervorrichtung für jeden der Verbraucher Daten empfängt, die eine tatsächliche Flüssigkeitsentnahmemenge angeben.

Die Daten über die Flüssigkeitsentnahmemenge können von dem jeweiligen Verbraucher bestimmt werden oder mittels eines externen Sensors. Die Daten können die Flüssigkeitsentnahmemenge enthalten oder derart ausgebildet sein, dass die Steuereinrichtung daraus die Flüssigkeitsentnahmemenge ableiten kann.

Gemäß der vorliegenden Offenbarung kann die Überwachung des tatsächlichen Ressourcenverbrauchs eine Bilanzierung von einem, in ein Ringsystem einströmenden Flüssigkeitsvolumen und aus dem Ringsystem ausströmenden Flüssigkeitsvolumen umfassen. Das Ringsystem kann beispielsweise eine Ringleitung umfassen, an die Komponenten des Dialysesystems angeschlossen sind.

Eine Bilanzierung kann umfassen, dass das gesamte einströmende Flüssigkeitsvolumen, beispielsweise zeitlich und/oder für verschiedene Zuflüsse integriert oder aufsummiert, und das gesamte ausströmende Flüssigkeitsvolumen, beispielsweise zeitlich und/oder für verschiedene Zuflüsse integriert oder aufsummiert, bestimmt werden. Zudem kann bestimmt werden, welche Differenz zwischen dem gesamten einströmenden Flüssigkeitsvolumen und dem gesamten ausströmenden Flüssigkeitsvolumen zu erwarten ist, beispielsweise anhand des insgesamt zu erwartenden Flüssigkeitsverbrauchs, bestimmt beispielsweise aus den Bedarfsdaten.

Die Bilanzierung kann zusätzlich oder alternativ auch für einen oder mehrere Teilabschnitte des Ringsystems durchgeführt werden.

Beispielsweise können an das Ringsystem eine oder mehrere Komponenten, beispielsweise die oben genannten Verbraucher, angeschlossen sein. Anhand des zu erwartenden Ressourcenverbrauchs, hier beispielsweise des zu erwartenden Flüssigkeitsverbrauchs, der Komponenten kann bestimmt werden, wie viel ausströmendes Flüssigkeitsvolumen bei einem bestimmten einströmenden Flüssigkeitsvolumen zu erwarten ist.

Wenn das tatsächlich ausströmende Flüssigkeitsvolumen geringer ist, als für das einströmende Flüssigkeitsvolumen erwartet, kann dies auf eine Leckage hindeuten.

Das Verfahren kann umfassen, dass eine Leckage im Dialysesystem erkannt wird, wenn die Abweichung des erwarteten und des tatsächlichen ausströmenden Flüssigkeitsvolumens außerhalb eines vorgegebenen Bereichs liegt. Insbesondere können dann das zu erwartende und das tatsächlich ausströmende Flüssigkeitsvolumen das oben genannte Parameterpaar darstellen.

Ein zu erwartendes aus dem Ringsystem ausströmendes Flüssigkeitsvolumen kann anhand der zu erwartenden Entnahmemengen der einzelnen an das Ringsystem angeschlossenen Komponenten bestimmt werden.

Eine Bilanzierung, insbesondere wenn diese nur für das gesamte Ringsystem und nicht für einzelne Teilabschnitte durchgeführt wird, erlaubt eine wenig aufwändige und dennoch zuverlässige Erkennung einer Leckage im System.

Hier sei angemerkt, dass das Erkennen einer Leckage gemäß der vorliegenden Erfindung sich auf das Vorliegen einer Leckage bezieht. Das Bestimmen, wo die Leckage auftritt, kann, muss aber nicht zwingend Teil des Verfahrens sein. Daher genügt die Bilanzierung zum Erkennen einer Leckage. Sollte das Verfahren auch das Bestimmen, wo die Leckage auftritt, umfassen, können dazu beispielsweise weitere Parameterpaare herangezogen werden, sofern die Bilanzierung dazu nicht ausreichen sollte.

Gemäß der vorliegenden Offenbarung kann das Verfahren umfassen, dass die Steuervorrichtung von einer oder mehreren Komponenten des Dialysesystems Messdaten zum einströmenden Flüssigkeitsvolumen und zum ausströmenden Flüssigkeitsvolumen empfängt und basierend darauf die Bilanzierung durchführt.

Gemäß der vorliegenden Offenbarung kann das Verfahren umfassen, dass die Steuereinrichtung Fehlermeldungen durch die (beispielsweise im Verbund betriebenen) Komponenten empfängt und auswertet. Beispielsweise kann eine der Komponenten ein Dialysegerät sein. Das Dialysegerät kann einen Feuchtesensor umfassen, der im Gehäuse des Dialysegeräts verbaut ist. Während der Heißreinigung kann das Dialysegerät mit einem Leckagealarm stehen bleiben. Die ausgetretene Feuchtmenge kann aber so gering sein, dass die zentrale Leckageüberwachung diese möglicherweise nicht zuverlässig/präzise detektieren würde. Das Dialysegerät kann seine Heißreinigung stoppen, was auch zur Folge haben kann, dass sich der zu erwartende Ressourcenverbrauch dementsprechend verringert.

Die Messdaten können beispielsweise mit Durchflussmessern bestimmt werden. Die Messdaten können durch die jeweilige Komponente selbst oder mittels nicht zur Komponente gehörenden Sensoren erfasst werden.

Gemäß der vorliegenden Offenbarung kann die Überwachung des tatsächlichen Ressourcenverbrauchs ein Überwachen einer zeitlichen Entwicklung des tatsächlichen Ressourcenverbrauchs umfassen und das Bestimmen einer Abweichung das Bestimmen einer Abweichung der zeitlichen Entwicklung des tatsächlichen Ressourcenverbrauchs von der zeitlichen Entwicklung des zu erwartenden Ressourcenverbrauchs umfassen.

Der Ressourcenverbrauch kann einen bestimmten Zeitverlauf aufweisen, insbesondere einen nicht-konstanten und insbesondere nicht-linearen Zeitverlauf. Der Zeitverlauf kann beispielsweise charakteristisch für einen bestimmten Betriebsmodus sein. Dieser Betriebsmodus kann beispielsweise jeweils von den Komponenten an die Steuervorrichtung gemeldet werden.

Die Steuervorrichtung kann dann die zeitliche Entwicklung als Indikator für das Vorliegen einer Leckage verwenden. Beispielsweise kann eine unerwartete Form im Zeitverlauf, insbesondere unerwartete Verbrauchsanstiege oder Spitzen, auf eine Leckage hindeuten.

Das Betrachten des Zeitverlaufs kann beispielsweise vorteilhaft sein, in Fällen, in denen Einzelwerte nicht ideal für eine zuverlässige Leckage-Erkennung sind und integrierte Werte aufgrund eines bestimmten Zeitverlaufs ebenfalls nicht die nötige Aussagekraft haben. In solchen Fällen kann der Zeitverlauf als solches ein geeigneter Indikator für das Vorliegen einer Leckage sein, optional in Kombination mit weiteren Parametern.

Gemäß der vorliegenden Offenbarung kann das Verfahren ein Empfangen, durch die Steuervorrichtung, von Daten von mindestens einem externen Informationssystem umfassen und das Erkennen der Leckage unter Berücksichtigung der von dem externen Informationssystem empfangenen Daten erfolgen.

Das externe Informationssystem kann als Informationssystem betrachtet werden, das nicht Teil des Dialysesystems ist. Beispielsweise kann es sich um ein Krankenhausinformationssystem (KIS) oder um ein Informationssystem, das Komponentenspezifikationen bereitstellt, handeln. Insbesondere kann das externe Informationssystem räumlich getrennt vom Dialysesystem, insbesondere außerhalb eines Dialysezentrums, angeordnet sein. Das Empfangen der Daten kann über gängige Datenschnittstellen erfolgen.

Es wird ermöglicht, beispielsweise von einem KIS oder einem Komponentenhersteller-Informationssystem Informationen zu erhalten, die für den Betrieb der (jeweiligen) Komponente relevant ist.

Das Berücksichtigen solcher Daten kann dazu genutzt werden, den zu erwartenden Ressourcenverbrauch bestimmter Komponenten, beispielsweise in bestimmten Betriebsmodi, zu bestimmen. Dies ermöglicht ein zuverlässiges Erkennen von Leckagen gemäß der vorliegenden Offenbarung.

Gemäß der vorliegenden Offenbarung kann das Verfahren einen durch die Steuervorrichtung veranlassten Austausch von Daten zwischen mindestens zwei der Komponenten mittelbar über die Steuervorrichtung umfassen.

Die Steuervorrichtung kann beispielsweise veranlassen, dass Daten, die die Steuervorrichtung von einer der Komponenten empfangen hat, an eine andere der Komponenten übertragen werden. Der durch die Steuervorrichtung veranlasste Austausch von Daten zwischen zwei Komponenten kann unidirektional oder bidirektional sein. Der durch die Steuervorrichtung veranlasste Austausch von Daten kann in Form eines Multicastings der Daten, die die Steuervorrichtung von einer der Komponenten empfangen hat, an mehrere der anderen Komponenten erfolgen. Insbesondere kann es sich bei den Daten um Komponenten-Betriebsdaten handeln. So kann beispielsweise ein Anschalten, ein Umstellen, ein Ausschalten und/oder eine Fehlfunktion einer Komponente an die Steuervorrichtung gemeldet werden und, über die Steuervorrichtung, mittelbar an eine oder mehrere andere Komponenten gemeldet werden.

Gemäß der vorliegenden Offenbarung kann der Ressourcenverbrauch einen Energieverbrauch und/oder einen Wasserverbrauch und/oder einen Zeitbedarf umfassen.

Der Energieverbrauch kann beispielsweise die Energiemenge sein, die zum Abschließen der Aufgabe erforderlich ist. Der Energieverbrauch kann dabei beispielsweise den Verbrauch elektrischer Energie und/oder von Energie in Form von Wärme umfassen. Der Wasserverbrauch kann beispielsweise die Wassermenge sein, die zum Abschließen der Aufgabe erforderlich ist. Bei dem Wasserverbrauch kann es sich um den Verbrauch von einer bestimmten Art von Wasser, beispielsweise aufbereitetem Wasser, handeln. Der Zeitbedarf kann beispielsweise die Zeitdauer sein, die zum Abschließen der Aufgabe erforderlich ist. Weitere denkbare Ressourcen sind dem Wasser zuzusetzende Inhaltsstoffe, beispielsweise Elektrolyte, oder andere Flüssigkeiten als Wasser.

Gemäß der vorliegenden Offenbarung können zumindest manche der Komponenten nur hydraulisch miteinander in Verbindung stehen.

Insbesondere können diese Komponenten keine direkte Datenverbindung aufweisen. Das heißt, diese Komponenten können nur mittelbar über die Steuervorrichtung Daten austauschen. Ein direkter Datenaustausch zwischen diesen Komponenten kann also nicht möglich sein. Dies kann eine verbesserte Sicherheit gewährleisten und außerdem ist es nicht erforderlich, kompatible Schnittstellen und Protokolle bereitzustellen.

Gemäß der vorliegenden Offenbarung können die Komponenten mindestens eine Umkehrosmose-Vorrichtung und/oder mindestens eine Konzentratmischanlage und/oder mindestens ein Heißreinigungssystem und/oder mindestens ein Dialysegerät umfassen.

Die Umkehrosmose-Vorrichtung kann zur (Dialyse-)Wasseraufbereitung ausgebildet sein. Die Umkehrosmose-Vorrichtung kann aufbereitetes Wasser als Ressource bereitstellen. Der Ressourcenverbrauch der Umkehrosmose-Vorrichtung kann, neben der Zeit, einen Wasserverbrauch, beispielsweise von verbrauchtem Wasser, und einen Energieverbrauch umfassen.

Die Konzentratmischanlage kann zum Anmischen von (Dialyse-)Konzentrat ausgebildet sein. Die Konzentratmischanlage kann Dialyse-Konzentrat als Ressource bereitstellen. Der Ressourcenverbrauch der Konzentratmischanlage kann, neben der Zeit, einen Wasserverbrauch, einen Energieverbrauch, einschließlich elektrischer Energie und Wärme, und Zusatz umfassen. Die Temperatur spielt dafür eine Rolle, wie schnell und wie gut das Konzentrat vermisch wird.

Das Heißreinigungssystem kann zum Heißreinigen von Behältern und Leitungen des Dialysesystems ausgebildet sein. Der Ressourcenverbrauch des Heißreinigungssystems kann, neben der Zeit, einen Wasserverbrauch und einen Energieverbrauch, einschließlich elektrischer Energie und Wärme, umfassen.

Das Dialysegerät kann zum Durchführen der Dialyse an einem Patienten ausgebildet sein. Der Ressourcenverbrauch des Dialysegeräts kann, neben der Zeit, einen Verbrauch von Dialyse-Konzentrat, einen Wasserverbrauch und einen Energieverbrauch, einschließlich elektrischer Energie und Wärme, umfassen. Das Dialysegerät kann verbrauchtes Wasser als Ressource zur Verfügung stellen.

Das Verfahren der vorliegenden Erfindung kann insbesondere vollständig automatisch, insbesondere durch die Steuervorrichtung, durchgeführt werden, soweit nicht explizit anders angegeben.

Die vorliegende Erfindung stellt auch ein Dialysesystem mit mehreren hydraulisch verbundenen Komponenten und einer Steuervorrichtung umfasst, wobei die Steuervorrichtung mit den Komponenten jeweils mittels einer Datenverbindung verbunden ist, wobei das Dialysesystem zur Durchführung des Verfahrens gemäß der vorliegenden Offenbarung ausgebildet ist.

Insbesondere kann die Datenverbindung eine Datenverbindung sein, die einen bi-direktionalen Datenaustausch ermöglicht, also insbesondere Übertragen von Daten von der Steuervorrichtung an die (jeweilige) Komponente und von der (jeweiligen) Komponente an die Steuervorrichtung.

Die Erfindung stellt auch ein das Dialysesystem umfassendes System bereit.

Das System kann auch ein vom Dialysesystem externes Informationssystem umfassen, beispielsweise ein Krankenhausinformationssystem (KIS) oder ein Informationssystem, das Komponentenspezifikationen bereitstellt. Insbesondere kann das externe Informationssystem räumlich getrennt vom Dialysesystem, insbesondere außerhalb eines Dialysezentrums, angeordnet sein.

Hinsichtlich der übrigen Merkmale und Vorteile wird auf die obigen Ausführungen verwiesen.

Die vorliegende Erfindung stellt auch ein Computerprogrammprodukt bereit, umfassend Befehle, die bewirken, dass das Dialysesystem der vorliegenden Offenbarung die Verfahrensschritte gemäß der vorliegenden Offenbarung, insbesondere wie oben beschrieben, ausführt.

Die vorliegende Erfindung stellt auch ein computerlesbares Medium bereit, auf dem das Computerprogrammprodukt der vorliegenden Offenbarung gespeichert ist.

Die Merkmale und Vorteile, die im Zusammenhang mit dem Verfahren beschrieben wurden, gelten entsprechend auch für das Dialysesystem, System, Computerprogrammprodukt und computerlesbare Medium.

Weitere Beispiele und Ausführungsformen werden im Folgenden anhand der Figuren erläutert. Dabei zeigen:
- Figur 1: eine schematische Darstellung eines Systems gemäß der vorliegenden Offenbarung;
- Figur 2: eine schematische Darstellung eines Verfahrens gemäß der vorliegenden Offenbarung;
- Figur 3: eine schematische Darstellung eines Systems gemäß der vorliegenden Offenbarung;
- Figur 4: eine schematische Darstellung eines Systems gemäß der vorliegenden Offenbarung.

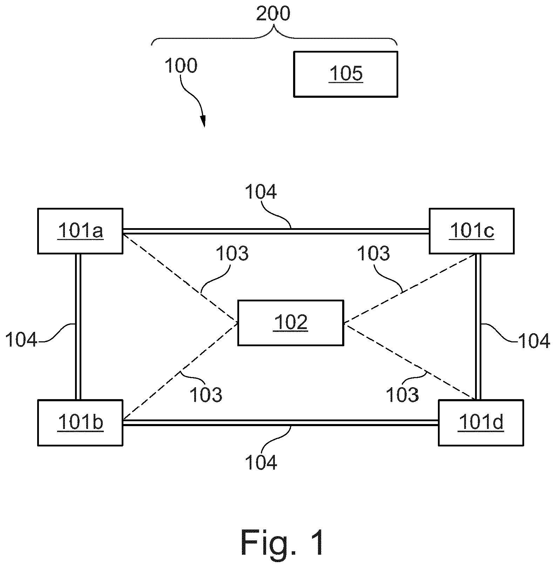

Figur 1 zeigt ein System 200 gemäß der vorliegenden Offenbarung umfassend ein Dialysesystem 100 gemäß der vorliegenden Offenbarung mit mehreren hydraulisch verbundenen Komponenten 101a, 101b, 101c, 101d und einer Steuervorrichtung 102, wobei die Steuervorrichtung mit den Komponenten jeweils mittels einer Datenverbindung 103, z.B. zum bi-direktionalen Datenaustausch, verbunden ist. Die hydraulische Verbindung ist mit dem Bezugszeichen 104 gekennzeichnet. Im vorliegenden Beispiel stehen die Komponenten nicht über eine Datenverbindung miteinander in Verbindung. Beispielsweise können sie ausschließlich hydraulisch miteinander verbunden sein.

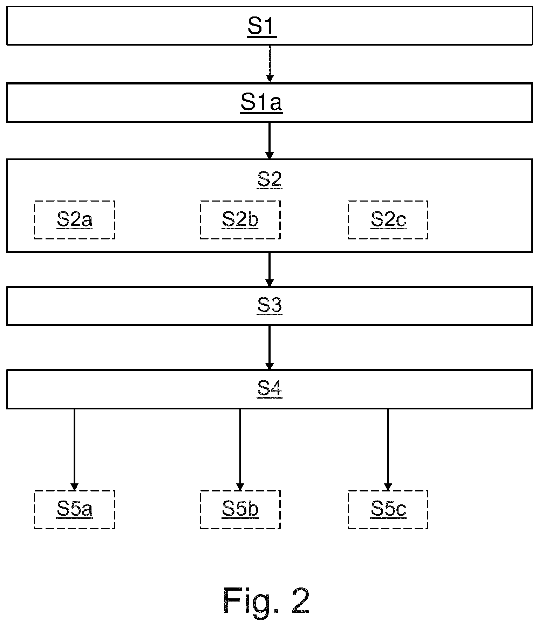

Das in Figur 1 gezeigte und oben beschriebene System kann zur Durchführung des Verfahrens gemäß der vorliegenden Offenbarung ausgebildet sein, insbesondere auch den im Zusammenhang mit Figuren 2 bis 4 beschriebenen Verfahrensschritten. Insbesondere kann die Steuervorrichtung ausgebildet sein, zumindest einen Teil der Verfahrensschritte, insbesondere alle Verfahrensschritte durchzuführen bzw. zu steuern.

Das System kann optional auch ein vom Dialysesystem externes Informationssystem 105 umfassen, beispielsweise ein Krankenhausinformationssystem (KIS) oder ein Informationssystem, das Komponentenspezifikationen bereitstellt. Insbesondere kann das externe Informationssystem räumlich getrennt vom Dialysesystem, insbesondere außerhalb eines Dialysezentrums, angeordnet sein.

In Figur 2 ist schematisch ein Verfahren zum Betreiben eines Dialysesystems, das mehrere hydraulisch miteinander in Verbindung stehende Komponenten, beispielsweise Medizinprodukte, und eine Steuervorrichtung umfasst, gemäß der vorliegenden Offenbarung dargestellt.

Das Verfahren umfasst, in Schritt S1, Empfangen von Komponenten-Betriebsdaten von den Komponenten durch die Steuervorrichtung. Die Komponenten-Betriebsdaten umfassend zumindest Bedarfsdaten für eine bevorstehende, durch die jeweilige Komponente auszuführende Aufgabe.

Das Verfahren umfasst, in Schritt S1a, eines zu erwartenden Ressourcenverbrauchs der (jeweiligen) Komponente anhand der Bedarfsdaten. Dabei handelt es sich beispielsweise um den zu erwartenden Ressourcenverbrauch für die bevorstehende, durch die Komponente auszuführende Aufgabe. Der Ressourcenverbrauch kann beispielsweise einen Energieverbrauch und/oder einen Wasserverbrauch und/oder einen Zeitbedarf umfassen.

Das Verfahren umfasst weiter, in Schritt S2, eine Überwachung eines tatsächlichen Ressourcenverbrauchs der Komponenten durch die Steuervorrichtung.

Die Überwachung des tatsächlichen Ressourcenverbrauchs kann beispielsweise ein Überwachen einer zeitlichen Entwicklung des tatsächlichen Ressourcenverbrauchs umfassen, im optionalen Schritt S2a.

Die Überwachung des tatsächlichen Ressourcenverbrauchs kann, wenn die Komponenten einen oder mehrere Verbraucher umfassen, im optionalen Schritt S2b umfassen, dass die Steuervorrichtung für jeden der Verbraucher Daten empfängt, die einen tatsächlichen Ressourcenverbrauch, beispielsweise eine tatsächliche Flüssigkeitsentnahmemenge, angeben.

Die Überwachung des tatsächlichen Ressourcenverbrauchs kann auch eine Bilanzierung von einem in ein Ringsystem einströmenden Flüssigkeitsvolumen und aus dem Ringsystem ausströmenden Flüssigkeitsvolumen umfassen, im optionalen Schritt S2c.

Das Verfahren umfasst weiter, in Schritt S3, ein Bestimmen einer Abweichung des tatsächlichen Ressourcenverbrauchs von dem zu erwartenden Ressourcenverbrauch durch die Steuervorrichtung.

Das Bestimmen einer Abweichung kann beispielsweise das Bestimmen einer Abweichung der oben erwähnten zeitlichen Entwicklung des tatsächlichen Ressourcenverbrauchs von der zeitlichen Entwicklung des zu erwartenden Ressourcenverbrauchs umfassen.

Das Verfahren umfasst weiter, in Schritt S4, ein Erkennen einer Leckage im Dialysesystem durch die Steuervorrichtung, wenn die Abweichung außerhalb eines vorgegebenen Bereichs liegt.

Das Erkennen der Leckage kann beispielsweise auch unter Berücksichtigung von einem externen Informationssystem empfangenen Daten erfolgen. Das Verfahren kann optional in Schritt S5a das Empfangen dieser Daten umfassen.

Das Verfahren kann umfassen, dass die Steuervorrichtung in Schritt S5b von einer oder mehreren Komponenten des Dialysesystems Messdaten zum einströmenden Flüssigkeitsvolumen und zum ausströmenden Flüssigkeitsvolumen empfängt. Diese Daten können beim Erkennen der Leckage verwendet werden. Insbesondere kann basierend auf diesen Daten eine Bilanzierung erfolgen.

Das Verfahren kann, in Schritt S5c, einen durch die Steuervorrichtung veranlassten Austausch von Daten zwischen mindestens zwei der Komponenten mittelbar über die Steuervorrichtung umfassen. Diese Daten können beim Erkennen der Leckage verwendet werden.

Weitere Ausführungsbeispiele und Vorteile von Verfahren bzw. Systemen gemäß der vorliegenden Offenbarung werden nachfolgend beschrieben.

Bei derzeitigen Verfahren und Systemen werden typischerweise an jedem einzelnen Medizinprodukt eines Dialysesystems Betriebsparameter eingestellt (beispielsweise ein Zeitfenster für eine jeweils geplante Aktion einprogrammiert) und, soweit möglich und gewünscht, gegebenenfalls die Komponente auf eine Leckage hin überwacht.

Bei derzeitigen Verfahren und Systemen kann keine systemweite bzw. das gesamte System abdeckende Leckage-Überwachung stattfinden, beispielsweise während der Entnahme von Wasser, da die Entnahmemengen der Verbraucher nicht bekannt sind.

Gemäß der vorliegenden Offenbarung können alle hydraulisch miteinander verbundenen Geräte bei der zentralen Steuerung ihre Bedarfe, beispielsweise für die dialysefreie Zeit, anmelden.

Komponenten des Dialysesystems können beispielsweise ihren jeweiligen Betriebsmodus an die Steuerung melden, aus denen die Steuerung die Bedarfe ableitet, oder aber unmittelbar Bedarfswerte bereitstellen. Weiter können die Komponenten oder andere Elemente des Dialysesystems Daten, aus denen tatsächliche Bedarfe hervorgehen, an die Steuerung melden. Die Steuerung kann dann Leckagen erkennen, wenn mehr als eine vorgegebene Abweichung tatsächlicher und erwarteter Bedarfe vorliegt.

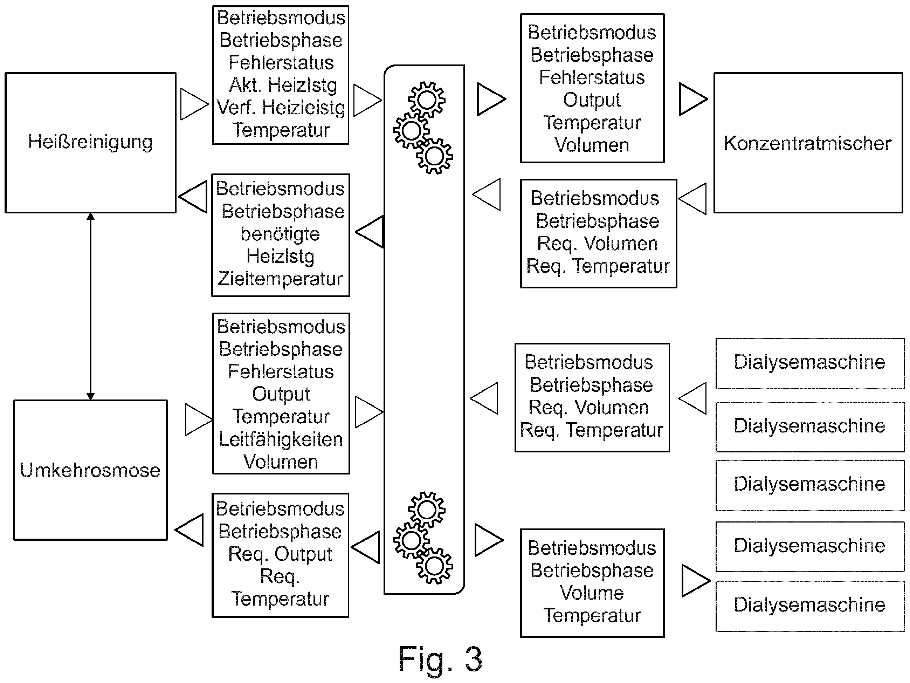

In Figur 3 ist eine schematische Darstellung der möglichen, in einem Dialysesystem gemäß der vorliegenden Offenbarung miteinander in Verbindung stehenden, aktiven Komponenten/Medizinprodukten gezeigt. Die zur zentralen Steuerung hinzeigenden Informationen werden von den angeschlossenen Komponenten/Medizinprodukten zur Verfügung gestellt. Die von der zentralen Steuerung herauszeigenden Information werden von der zentralen Steuerung an Komponenten bereitgestellt.

Insbesondere sind hier die Komponenten "Heißreinigung", "Umkehrosmose", "Konzentratmischer" und "Dialysemaschine" beispielhaft gezeigt.

Die Komponenten können jeweils Betriebsmodus und Betriebsphase an die Steuerung melden. Zudem können auch Fehlerstatus, aktuelle Heizleistung (AKT HEIZLSTG, bspw. in kW), verfügbare Heizleistung (VERF HEIZLSTG, bspw. in kW) und Temperatur von der Heißreinigung gemeldet werden. Von der Umkehrosmose können zudem Fehlerstatus, Output (beispielsweise in Liter/Stunde), Temperatur, Leitfähigkeiten und Volumen gemeldet werden. Vom Konzentratmischer und den Dialysemaschinen können zudem jeweils Volumenbedarf (REQ Volumen), beispielsweise an Wasser von der Umkehrosmose, und Temperaturbedarf (REQ Temperatur), beispielsweise Temperatur des Wassers aus der Umkehrosmose kommend, gemeldet werden.

Von der zentralen Steuerung können dann beispielsweise Betriebsmodus, Betriebsphase, Fehlerstatus, Output, Temperatur, Volumen an die Komponenten gemeldet werden. Außerdem können benötigte Heizleistung und Zieltemperatur an die Heißreinigung und/oder Volumenbedarf (REQ Volumen) und Temperaturbedarf (REQ Temperatur) an die Umkehrosmose gemeldet werden.

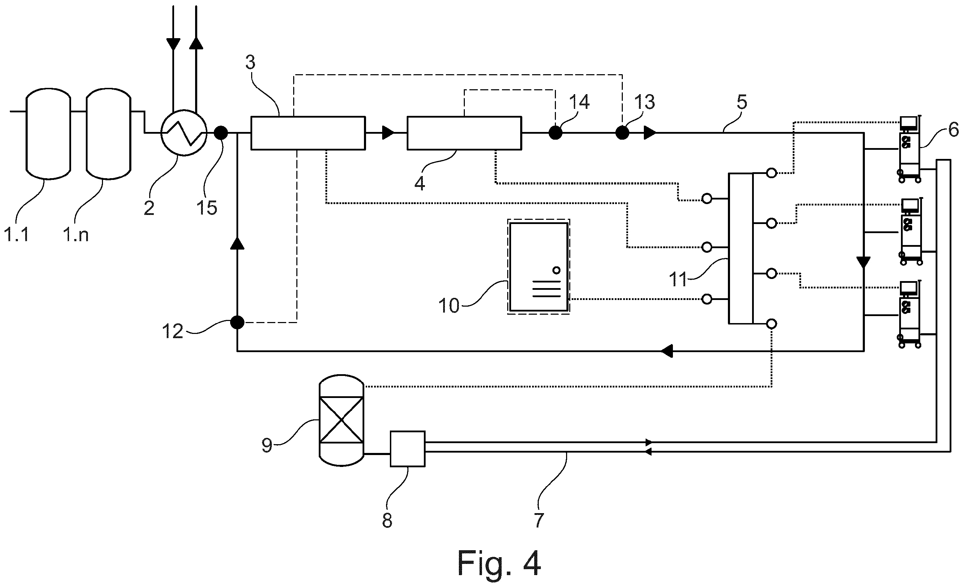

In Figur 4 ist eine schematische Darstellung der hydraulischen und informationstechnischen Verbindungen in einem beispielhaften Dialysezentrum umfassend ein beispielhaftes Dialysesystem gemäß der vorliegenden Offenbarung gezeigt. Das Dialysesystem umfasst in der Figur beispielhaft die folgenden Komponenten:
- 1.1: Vorbehandlungseinheit 1
- 1.n: Vorbehandlungseinheit n
- 2: Wärmetauscher
- 3: Umkehrosmose
- 4: Heißreinigung
- 5: Permeatringleitung
- 6: Verbraucher / Dialysegerät
- 7: Konzentratringleitung
- 8: Konzentratverteilanlage
- 9: Konzentratmischer
- 10: Steuerung
- 11: Netzwerk
- 12: Durchflußmesser Austritt Permeatringleitung
- 13: Durchflußmesser Eintritt Permeatringleitung
- 14: Temperatursensor Heißreinigung
- 15: Temperatursensor Austritt Wärmetauscher

Keines der oben gennannten Elemente, mit Ausnahme der Steuerung und ggf. geeigneter Datenverbindungen, ist zwingend erforderlich. Untergruppen der genannten Elemente können miteinander geeignet kombiniert werden. Insbesondere kann das Dialysesystem ohne Konzentratmischer 9 ausgebildet sein. Das Dialysesystem kann auch ohne Vorschaltung des Wärmetauschers sowie ohne den Temperatursensor Austritt Wärmetauscher 15 ausgebildet sein.

Im Folgenden wird ein beispielhaftes Verfahren gemäß der vorliegenden Offenbarung beschrieben. Bei der Beschreibung des Verfahrens wird rein beispielhaft auf das Dialysesystem, das in Figur 4 gezeigt ist, Bezug genommen. Das Verfahren kann aber auch mittels anderer geeigneter Systeme durchgeführt werden. Gemäß dem Verfahren können alle über ein Netzwerk 11 mit einer Steuerung 10 verbundenen Komponenten (Medizinprodukte) ihre Bedarfe, beispielsweise Volumen, Temperatur, Betriebsmodus und/oder Betriebsphase, zentral bei der Steuerung anmelden.

Nach Ermittlung der Ressourcenbedarfe, beispielsweise Permeatvolumen-Bedarfe, Temperaturen-Bedarfe, und/oder Zeitbedarfe, aller angeschlossenen Komponenten/Medizinprodukte kann die Steuerung eine Leckageerkennung gemäß der vorliegenden Offenbarung durchführen.

Beispielsweise kann aufgrund der angemeldeten Bedarfe der verbundenen Verbraucher durch eine Bilanzierung der Volumenströme am Eingang 13 und am Austritt 12 der Permeatringleitung 5 eine Leckageüberwachung ermöglicht werden.

So kann die Bilanzierung umfassen, dass bestimmt wird, welcher Volumenstrom am Austritt bei einem Bestimmten Volumenstrom am Eingang erwartet wird, wenn die tatsächlichen Bedarfe der verbundenen Verbraucher den angemeldeten Bedarfen entsprechen. Wenn dann am Austritt der tatsächliche Volumenstrom um mehr als einen vorgegebenen Wert von dem erwarteten Volumenstrom abweicht, kann die Steuerung bestimmen, dass eine Leckage vorliegt.

Obwohl die Erfindung in den Figuren und der vorstehenden Beschreibung detailliert dargestellt und beschrieben ist, sind diese Figuren und Beschreibungen als beispielhaft und nicht einschränkend zu betrachten. Die Erfindung ist nicht auf die gezeigten Ausführungsformen beschränkt. In Anbetracht der vorstehenden Beschreibung und der Figuren wird es für den Fachmann offensichtlich sein, dass im Rahmen der Erfindung, wie sie in den Ansprüchen definiert ist, verschiedene Modifikationen vorgenommen werden können.

## Patentansprüche

1. Verfahren zum Betreiben eines Dialysesystems (100) umfassend mehrere hydraulisch miteinander in Verbindung stehende Komponenten (101a, 101b, 101c, 101d) und eine Steuervorrichtung (102), wobei das Verfahren umfasst,
Empfangen (S1), durch die Steuervorrichtung, von Komponenten-Betriebsdaten von den Komponenten, wobei die Komponenten-Betriebsdaten zumindest Bedarfsdaten für eine bevorstehende, durch die jeweilige Komponente auszuführende Aufgabe umfassen,
Bestimmen (S1a) eines zu erwartenden Ressourcenverbrauchs der Komponente anhand der Bedarfsdaten,
Überwachung (S2), durch die Steuervorrichtung, eines tatsächlichen Ressourcenverbrauchs der Komponenten,
Bestimmen (S3), durch die Steuervorrichtung, einer Abweichung des tatsächlichen Ressourcenverbrauchs, von dem zu erwartenden Ressourcenverbrauch, und
Erkennen (S4), durch die Steuervorrichtung, einer Leckage im Dialysesystem, wenn die Abweichung außerhalb eines vorgegebenen Bereichs liegt.

2. Verfahren nach Anspruch 1, wobei der zu erwartende Ressourcenverbrauch durch einen oder mehrere Bedarfsparameter repräsentiert wird und der tatsächliche Ressourcenverbrauch durch einen oder mehrere Verbrauchsparameter repräsentiert wird, wobei jeweils ein Bedarfsparameter und ein Verbrauchsparameter ein Parameterpaar bilden und die Abweichung für mindestens eines der Parameterpaare bestimmt wird.

3. Verfahren nach Anspruch 2, wobei für mehrere, insbesondere alle, Parameterpaare jeweils eine Abweichung des tatsächlichen Ressourcenverbrauchs von dem zu erwartenden Ressourcenverbrauch bestimmt wird und eine Leckage erkannt wird, wenn mindestens eine vorgegebene Anzahl der Abweichungen außerhalb eines für das Parameterpaar vorgegebenen Bereichs liegt.

4. Verfahren nach Anspruch 2, wobei für mehrere, insbesondere alle, Parameterpaare jeweils eine Abweichung des tatsächlichen Ressourcenverbrauchs von dem zu erwartenden Ressourcenverbrauch bestimmt wird und eine Leckage erkannt wird, wenn mindestens eine der Abweichungen außerhalb eines für das Parameterpaar vorgegebenen Bereichs liegt.

5. Verfahren nach einem der vorangegangenen Ansprüche, wobei die Komponenten einen oder mehrere Verbraucher umfassen und wobei die Überwachung des tatsächlichen Ressourcenverbrauchs umfasst, dass die Steuervorrichtung für jeden der Verbraucher Daten empfängt, die eine tatsächliche Flüssigkeitsentnahmemenge angeben.

6. Verfahren nach einem der vorangegangenen Ansprüche, wobei die Überwachung des tatsächlichen Ressourcenverbrauchs eine Bilanzierung von einem in ein Ringsystem einströmenden Flüssigkeitsvolumen und aus dem Ringsystem ausströmenden Flüssigkeitsvolumen umfasst.

7. Verfahren nach Anspruch 6, wobei das Verfahren umfasst, dass die Steuervorrichtung von einer oder mehreren Komponenten des Dialysesystems Messdaten zum einströmenden Flüssigkeitsvolumen und zum ausströmenden Flüssigkeitsvolumen empfängt und basierend darauf die Bilanzierung durchführt.

8. Verfahren nach einem der vorangegangenen Ansprüche, wobei die Überwachung des tatsächlichen Ressourcenverbrauchs ein Überwachen einer zeitlichen Entwicklung des tatsächlichen Ressourcenverbrauchs umfasst und wobei das Bestimmen einer Abweichung das Bestimmen einer Abweichung der zeitlichen Entwicklung des tatsächlichen Ressourcenverbrauchs von der zeitlichen Entwicklung des zu erwartenden Ressourcenverbrauchs umfasst.

9. Verfahren nach einem der vorangegangenen Ansprüche, wobei das Verfahren ein Empfangen, durch die Steuervorrichtung, von Daten von mindestens einem externen Informationssystem umfasst und das Erkennen der Leckage unter Berücksichtigung der von dem externen Informationssystem empfangenen Daten erfolgt.

10. Verfahren nach einem der vorangegangenen Ansprüche, umfassend einen durch die Steuervorrichtung veranlassten Austausch von Daten zwischen mindestens zwei der Komponenten mittelbar über die Steuervorrichtung.

11. Verfahren nach einem der vorangegangenen Ansprüche, wobei zumindest manche der Komponenten nur hydraulisch miteinander in Verbindung stehen.

12. Verfahren nach einem der vorangegangenen Ansprüche, wobei die Komponenten mindestens eine Umkehrosmose-Vorrichtung und/oder mindestens eine Konzentratmischanlage und/oder mindestens ein Heißreinigungssystem und/oder mindestens ein Dialysegerät umfassen.

13. Dialysesystem (100) mit mehreren hydraulisch verbundenen Komponenten (101a, 101b, 101c, 101d) und einer Steuervorrichtung, wobei die Steuervorrichtung (102) mit den Komponenten jeweils mittels einer Datenverbindung verbunden ist, wobei das Dialysesystem zur Durchführung des Verfahrens nach einem der vorangegangen Ansprüche ausgebildet ist.

14. Computerprogrammprodukt, umfassend Befehle, die bewirken, dass das Dialysesystem des Anspruchs 13 die Verfahrensschritte nach einem der Ansprüche 1 bis 12 ausführt.

15. Computerlesbares Medium, auf dem das Computerprogramm nach Anspruch 14 gespeichert ist.
